# EUROPÄISCHE PATENTSCHRIFT

(11) **EP 0 961 621 B1**
(45) Veröffentlichungstag und Bekanntmachung des Hinweises auf die Patenterteilung: **07.07.2004**
(21) Anmeldenummer: 97918098.1
(22) Anmeldetag: 02.04.1997
(51) Int. Cl.: A61M 1/10

(54) **INTRAVASALE BLUTPUMPE**
INTRAVASCULAR BLOOD PUMP
POMPE A SANG INTRAVASCULAIRE

(30) Priorität: 04.04.1996 DE 19613564
(43) Veröffentlichungstag der Anmeldung: 08.12.1999
(73) Patentinhaber: Impella CardioSystems AG, 52074 Aachen (DE)
(72) Erfinder: Impella CardioSystems AG, 52074 Aachen (DE)
(74) Vertreter: Selting, Günther, Dipl.-Ing.
(86) Internationale Anmeldenummer: PCT/EP1997/001652
(87) Internationale Veröffentlichungsnummer: WO 1997/037696

(56) Entgegenhaltungen:
- EP-A- 0 629 412
- WO-A-94/09835
- SIESS ET AL: 'Hydraulic refinement of an intraarterial microaxial blood pump' INT. J. OF ARTIFICIAL ORGANS Bd. 18, Nr. 5, Seiten 273 - 285

## Beschreibung

Die Erfindung betrifft eine intravasale Blutpumpe mit einem Antriebsteil und einem Pumpenteil, der durch das Gefäßsystem des menschlichen Körpers hindurchgeführt werden kann, um beispielsweise im Herzen Pumparbeit auszuführen.

Eine intravasale Blutpumpe wird durch Punktion eines Blutgefäßes in das Gefäßsystem des Körpers eingeführt und zum Herzen oder an eine andere Stelle, an der Blut gepumpt werden soll, vorgeschoben. Diejenigen Teile, die in den Körper eingeführt werden, müssen im Durchmesser so klein sein, daß sie durch die extern zugänglichen, großen Gefäße hindurchpassen. Der größte zulässige Durchmesser liegt bei etwa 7 mm.

Aus EP 0 157 871 B1 und EP 0 397 668 B1 sind intravasale Blutpumpen bekannt, bei denen der Pumpenteil ein rohrförmiges Gehäuse enthält, in dem ein Flügelrad drehbar angeordnet ist. Das Flügelrad ist über eine flexible Welle, die durch einen Katheter hindurch verläuft, mit einem extrakorporalen Antriebsteil verbunden. Der Antriebsteil treibt die flexible Welle an, die ihrerseits den Pumpenteil antreibt. Dabei kann der Antriebsteil wegen seines extrakorporalen Betriebs beliebig groß ausgebildet sein. Zur Herabsetzung der Reibung zwischen Welle und Katheter ist eine kontinuierliche Schmierung mit Flüssigkeit erforderlich. Ein Teil dieser Flüssigkeit gelangt durch die Gleitlager und Dichtung der Pumpeneinheit samt Abrieb in den Blutstrom. Der andere Teil wird nach der Passage durch den Katheter entlang der Welle extrakorporal gesammelt. Ferner schränkt die flexible Welle die Einsatzmöglichkeiten der Blutpumpe ein, weil diese nur zu solchen Stellen im Körper gebracht werden kann, bei denen keine allzu starken Biegungen des Katheters und der darin enthaltenen Welle erforderlich sind.

Aus WO94/09835 ist eine Blutpumpe für die vorübergehende Herzunterstützung .bekannt. Diese Blutpumpe, die am operativ geöffneten Herzen eingesetzt wird, weist ein stabförmiges Gehäuse auf, das den Motor und die Pumpe enthält und mit seinem Pumpenteil in die Aorta eingesetzt werden kann, während der Motorteil.außerhalb der Aorta verbleibt. Eine das Flügelrad tragende Motorwelle ist in vier Lagern gelagert, nämlich in zwei Lagern, die an beiden Enden des Motorgehäuses angeordnet sind und einem dritten und vierten Lager, die am flügelradseitigen Ende des stationären Nabenteils angeordnet sind. Wegen der vier Lager hat die Blutpumpe insgesamt eine große Länge. Wegen dieser großen Länge und wegen des großen Durchmessers ist die Pumpe als intravasale Blutpumpe nicht geeignet.

EP 0 629 412 A2 beschreibt eine Blutpumpe, die operativ in das Herz eingeführt wird. Von einem Antriebsteil mit großem Durchmesser steht ein Pumpenteil von kleinerem Durchmesser ab. Die Motorwelle ist an zwei Stellen gelagert, nämlich an dem vorderen Ende des Motorgehäuses und an dem flügelradseitigen Ende eines stationären Nabenteils. Diese Pumpenstruktur kommt aufgrund des Motors mit im Vergleich zum Pumpengehäuse deutlich größerem Durchmesser für eine intravasale Blutpumpe nicht in Betracht.

Ferner ist durch EP 0 157 859 B1 eine Blutpumpe bekannt, bei der der Motorteil und der Pumpenteil baulich vereinigt sind. Diese Pumpe ist implantierbar, jedoch handelt es sich nicht um eine intravasale Blutpumpe, die minimalinvasiv in den Körper eingeführt werden kann.

Das Konzept einer intravasalen Blutpumpe, von der der Oberbegriff des Patentanspruchs 1 ausgeht, ist beschrieben in T. Sieß u.a., "Hydraulic refinement of an intraarterial microaxial blood pump" in "The International Journal of Artificial Organs" / Vol. 18 / Nr. 5, 1995, S. 273-285. Die dort beschriebene Blutpumpe weist ein einen Elektromotor enthaltendes Motorgehäuse auf, das am rückwärtigen Ende mit einem Katheter verbunden ist. In dem Motorgehäuse ist die Motorwelle an zwei Enden gelagert. Von dem vorderen Ende des Motorgehäuses steht ein Rohr ab, durch das die Motorwelle hindurchragt. An diesem Rohr ist ein rohrförmiges Pumpengehäuse befestigt, in welchem das mit der Motorwelle verbundene Flügelrad rotiert. Das Pumpengehäuse und das Motorgehäuse haben etwa gleichen Durchmesser, der 5,4 bis 6,4 mm betragen kann. Bei dieser intravasalen Blutpumpe kann eine genaue Zentrierung des Flügelrades in dem Pumpengehäuse nur sehr schwer erreicht werden. Geringe Zentrierungsfehler führen bei den geringen Abmessungen bereits zu einer Verschlechterung des Rundlaufes und können schwerwiegende Blutschädigungen zur Folge haben.

Der Erfindung liegt die Aufgabe zugrunde, eine intravasale Blutpumpe zu schaffen, also eine Blutpumpe, die durch Blutgefäße hindurch vorgeschoben werden kann, welche bei der erforderlichen großen Pumpenleistung kleinformatig ausgebildet ist, und bei der die Blutschädigung gering ist.

Die Lösung dieser Aufgabe erfolgt erfindungsgemäß mit den im Patentanspruch 1 angegebenen Merkmalen.

Bei der erfindungsgemäßen Blutpumpe sind Antriebsteil und Pumpenteil unmittelbar miteinander verbunden und die Blutpumpe ist als stabförmiges dünnes Teil ausgeführt, wobei Motorgehäuse und Pumpengehäuse im wesentlichen gleiche Außendurchmesser haben. Das Flügelrad sitzt auf der Motorwelle, die sich durch ein von dem Motorgehäuse abstehendes Nabenteil erstreckt, an dessen Ende sie gelagert ist.

Die das Flügelrad tragende Motorwelle erstreckt sich durch das Motorgehäuse hindurch und ist an ihren beiden Enden gelagert, so daß sie eine gute Führung und Konstanz ihrer axialen Ausrichtung hat. Dies ist Voraussetzung für eine schlagfreie zentrisch genaue Führung des Flügelrades. Eine derartig genaue Führung ist erforderlich, da zur Minimierung der Blutschädigung und zur Vermeidung hydraulischer Leistungsverluste der Spalt zwischen den Flügeln des Flügelrades und dem Pumpengehäuse 1/10 mm nicht übersteigen sollte. Unrundheiten der Flügelraddrehung würden ebenfalls die Gefahr der Hämolyse vergrößern. Durch die Lagerung der Motorwelle an beiden voneinander entfernt liegenden Enden und in unmittelbarer Nähe des Flügelrades wird die Lagergenauigkeit erhöht, so daß Blutschädigungen durch exzentrische Anordnung des Flügelrades und durch schlechten Rundlauf vermieden werden.

Das flügelradseitige Lager ist vorzugsweise zugleich als Wellendichtung ausgebildet, wodurch verhindert wird, daß Blut in das Rohr oder das Pumpengehäuse eintritt.

Der Durchmesser einer minimalinvasiv plazierbaren Blutpumpe ist auf etwa 5 bis 7 mm beschränkt, da die Gefäßweite in den Körperrandbereichen maximal etwas mehr als 7 mm beträgt. Mit einer derartigen Blutpumpe kann eine Pumpenleistung von ca. 4 l/min. bei etwa 100 mmHg Gegendruck erzeugt werden.

Die erfindungsgemäße intravasale Blutpumpe ist wegen ihrer guten Beweglichkeit im Gefäßsystem vielseitig einsetzbar, beispielsweise
a) als Linksherzunterstützungspumpe mit der Option (siehe b)) zur Erzeugung einer pulsierenden Strömung,
b) als Rechtsherzunterstützungspumpe mit der Option des pulsatilen Betriebs durch Modulation der Pumpendrehzahl,
c) als uni- oder biventrikuläres Unterstützungssystem während thorakaler/transthorakaler Operationen am schlagenden oder nicht schlagenden Herzen ohne den Einsatz einer Herz-Lungen-Maschine,
d) als Blutpumpe zur lokalen Organperfusion mit entsprechender Dichteinrichtung.

Da der Pumpenteil nach einem Einsatz der Blutpumpe nur schwer zu reinigen und zu sterilisieren ist, ist gemäß einer bevorzugten Weiterbildung der Erfindung vorgesehen, daß das Pumpengehäuse, das Flügelrad und der Schaft eine Wegwerfeinheit bilden, die als Ganzes von dem Motorgehäuse entfernbar ist. Zu dieser Wegwerfeinheit kann auch noch der Rotor der Antriebseinheit gehören, wenn dieser fest mit dem Schaft verbunden ist. Auf diese Weise ist es möglich, diejenigen Teile, die ohne größeren Aufwand hergestellt werden können, in der Wegwerfeinheit zu vereinigen, während der Stator des Motors, der teure Wicklungen erfordert und ein leicht zu sterilisierendes Gehäuse aufweist, mehrfach benutzt werden kann.

Im folgenden werden unter Bezugnahme auf die Zeichnungen Ausführungsbeispiele der Erfindung näher erläutert.

Es zeigen:
- Fig. 1: eine schematische Darstellung der Einführung der Blutpumpe vor den linken Ventrikel, mit Positionierung der Ansaugkanüle im linken Ventrikel,
- Fig. 2: einen schematischen Längsschnitt eines ersten Ausführungsbeispiels der Blutpumpe,
- Fig. 3: eine vergrößerte Darstellung der Einzelheit III aus Fig. 2,
- Fig. 4: ein zweites Ausführungsbeispiel, bei dem ein Teil der Blutpumpe als Wegwerfeinheit ausgebildet ist, und
- Fig. 5: ein drittes Ausführungsbeispiel, bei dem das Pumpengehäuse mit einem Verbindungssteg mit dem Motorgehäuse verbunden ist.

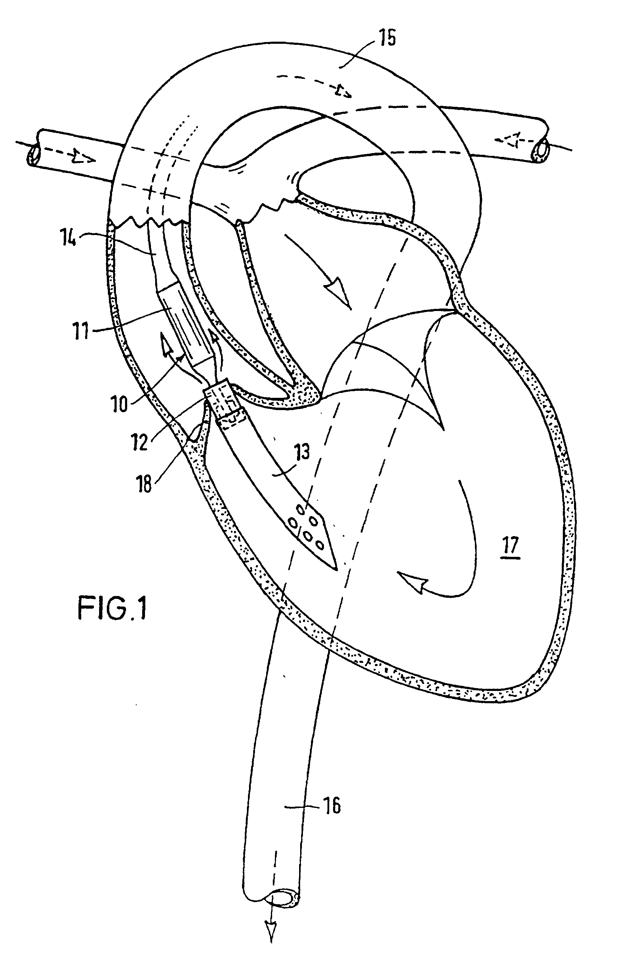

In Fig. 1 ist eine Verwendung der Blutpumpe 10 zur Linksherzunterstützung dargestellt. Die Blutpumpe 10 weist einen Motorteil 11 und einen Pumpenteil 12 auf, die koaxial hintereinander angeordnet sind und eine stabförmige Bauform ergeben. Der Pumpenteil ist durch einen Ansaugschlauch 13 verlängert, der an seinem Ende und/oder in seiner Seitenwand Öffnungen für den Blutzutritt zur Pumpe aufweist. Das dem Saugschlauch 13 abgewandte rückwärtige Ende der Blutpumpe 10 ist mit einem Katheter 14 verbunden, der durch den Aortenbogen 15 und die Aorta 16 eingeführt wurde. Die Blutpumpe 10 wird so plaziert, daß sie hauptsächlich in der aufsteigenden Aorta 15 liegt, der gerade und kurze Saugschlauch 13 aber in die Herzkammer 17 hineinragt. Die Aortenklappe 18 legt sich im Schließzustand an die Außenseite des Pumpengehäuses oder des Saugschlauchs. Die Blutpumpe 10 mit vorgelagertem Saugschlauch 13 wird durch Vorschieben des Katheters 14, ggf. mit einem darin enthaltenen Mandrin oder unter Verwendung eines Führungsdrahtes, in die dargestellte Position vorgeschoben. Der Saugschlauch 13 passiert dabei die Aortenklappe 18 retrograd, so daß durch den Saugschlauch 13 Blut angesaugt und in die Aorta 16 gepumpt wird.

Der Einsatz der erfindungsgemäßen Blutpumpe ist nicht auf die in Fig. 1 dargestellte Anwendung, bei der es sich lediglich um ein typisches Beispiel handelt, beschränkt.

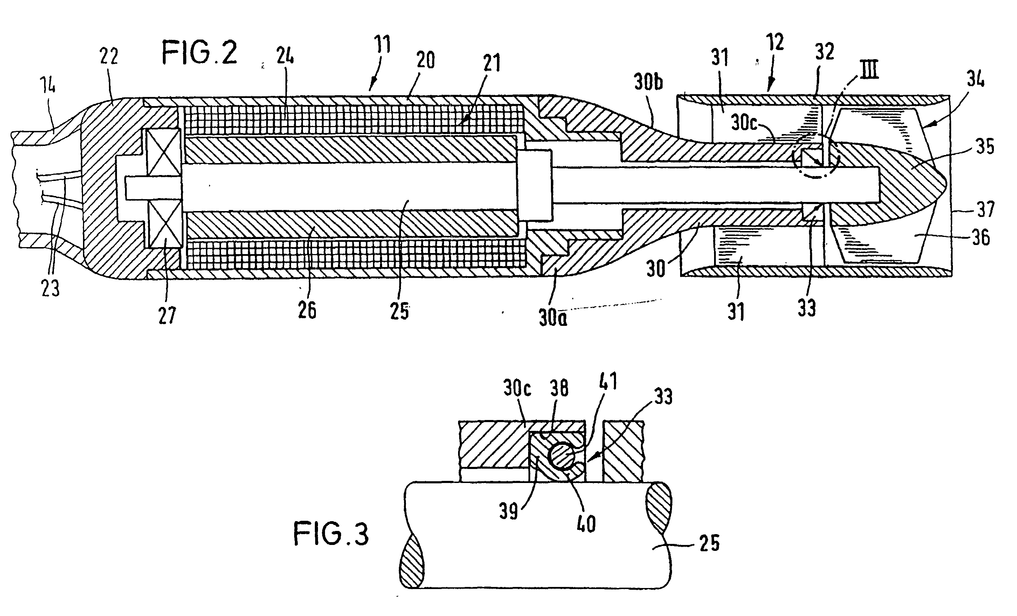

Fig. 2 zeigt ein bevorzugtes Ausführungsbeispiel der Blutpumpe mit dem Motorteil 11 und dem damit fest verbundenen Pumpenteil 12. Der Motorteil 11 weist ein langgestrecktes zylindrisches Gehäuse 20 auf, in dem der Elektromotor 21 untergebracht ist. An dem rückwärtigen Ende ist das Gehäuse 20 mit einer Stirnwand 22 verschlossen, an die sich der flexible Katheter 14 abdichtend anschließt. Durch den Katheter 14 verlaufen die elektrischen Kabel 23 zur Stromversorgung und Steuerung des Elektromotors 21.

Der Stator 24 des Motors weist in üblicher Weise zahlreiche umfangsmäßig verteilt angeordnete Wicklungen, sowie einen magnetischen Rückschluß in Längsrichtung auf. Er ist mit dem Motorgehäuse 20 fest verbunden. Der Stator 24 umgibt den mit der Motorwelle 25 verbundenen Rotor 26, der aus in Wirkrichtung magnetisierten Permanentmagneten besteht. Die Motorwelle ist am rückwärtigen Ende mit einem Lager 27 im Motorgehäuse bzw. in der Stirnwand 22 gelagert. Sie erstreckt sich durch die gesamte Länge des Motorgehäuses 20 und ragt nach vorne aus diesem heraus.

Den vorderen Abschluß des Motorgehäuses 20 bildet ein rohrförmiges stationäres Nabenteil 30, das am rückwärtigen Ende 30a den gleichen Durchmesser hat wie das Motorgehäuse 20 und abdichtend mit dem Motorgehäuse verbunden ist. An das rückwärtige Ende 30a schließt sich ein Verjüngungsabschnitt 30b an, in welchem sich der Außendurchmesser des Nabenteils 30 stetig verringert bis zum zylindrischen vorderen Endabschnitt 30c. Von dem Endabschnitt 30c des Rohres 30 stehen radiale Rippen 31 ab, deren äußere Enden mit dem zylindrischen rohrförmigen Pumpengehäuse 32 verbunden sind. Am vorderen Ende des Endabschnitts 30c befindet sich im Rohr 30 ein Lager 33 zur Lagerung der Motorwelle 25. Dieses Lager ist zugleich als Wellendichtung ausgebildet und wird später noch erläutert.

Die Motorwelle 25 steht aus dem Rohr 30 nach vorne vor und trägt dort ein Flügelrad 34 mit einer auf dem Schaftende sitzenden Nabe 35 und davon abstehenden Flügeln 36 oder Pumpenschaufeln.

Bei einer Rotation des Flügelrades 34 wird Blut durch die stirnseitige Ansaugöffnung 37 des Pumpengehäuses 32 angesaugt und in axialer Richtung im Pumpengehäuse 32 nach hinten getrieben. Durch den ringförmigen Spalt zwischen dem Pumpengehäuse 32 und dem Motorgehäuse 20 strömt das Blut entlang des Abschnitts 30b des Rohres 30 nach außen, um weiter an dem Motorgehäuse 20 entlangzuströmen. Hierdurch wird der Abtransport der im Antrieb erzeugten Wärme sichergestellt, ohne daß es zu Blutschädigung durch zu hohe Oberflächentemperaturen (über 41 °C) auf dem Motorgehäuse 20 kommt. Motorgehäuse 20 und Pumpengehäuse 32 haben etwa den gleichen Durchmesser, jedoch kann der Außendurchmesser des Pumpengehäuses 32 etwas größer sein als derjenige des Motorgehäuses, weil das Pumpengehäuse außen nicht umströmt werden muß. Es ist auch möglich, bei diesem und den nachfolgenden Beispielen, den Pumpenteil mit umgekehrter Förderrichtung zu betreiben, wobei Blut an dem Motorgehäuse entlang angesaugt wird und aus dem Ende 37 axial austritt.

Das Lager 33 sitzt in einer nach innen und zum Ende hin offenen Ausnehmung 38 am äußersten vorderen Ende des Nabenteils 30. Es weist einen Lagerkörper 39 aus Polytetrafluorethylen auf, das eine große Härte und einen niedrigen Reibungskoeffizienten mit Stahl hat. Dieser Lagerkörper weist an seiner Innenseite einen Dichtwulst 40 auf, der die rotierende Motorwelle 25 nahezu linienförmig umgibt. Im Innern des Lagerteils 39 befindet sich eine Spreizfeder 41, die den Wulstbereich 40 mit Kraft gegen den Schaft 25 drückt.

Durch die Lager 27 und 33 an den äußersten Enden der Motorwelle 25 wird eine exakte Führung und ein guter Rundlauf der Motorwelle erreicht. Vorteilhaft ist insbesondere, daß das vordere Lager 33 sich sehr nahe an dem Flügelrad 34 befindet.

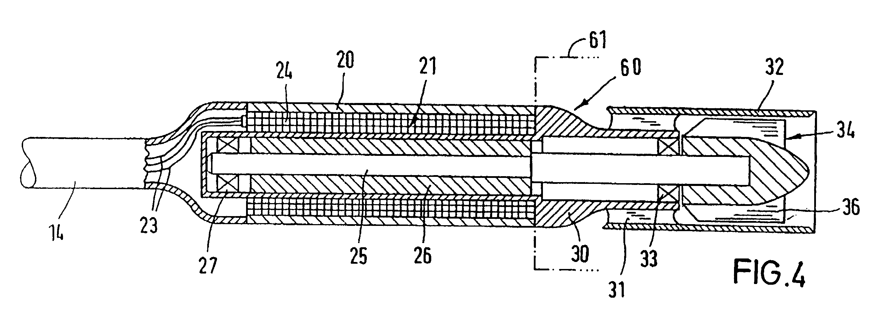

Das Ausführungsbeispiel von Fig. 4 gleicht weitgehend demjenigen von Fig. 2, so daß die nachfolgende Beschreibung sich auf die Erläuterung der Unterschiede beschränkt. Das Flügelrad 34 bildet zusammen mit der Motorwelle 25 und dem Rotor 26 sowie dem Rohr 30 und dem Pumpengehäuse 32 eine Wegwerfeinheit, deren Grenzen durch die gestrichelte Linie 61 bezeichnet sind. Zu der Wegwerfeinheit gehört auch das Lager 33 und ggf. das Lager 27. Nach Gebrauch der Blutpumpe können die genannten Teile kontaminiert sein, wobei sich auch Blutpartikel in Spalten festsetzen können. Daher ist eine vollständige Entkeimung praktisch nicht möglich. Die Wegwerfeinheit 60 besteht aus Teilen, die relativ einfach und kostengünstig hergestellt werden können. Sie wird als Ganzes von dem Motorgehäuse 20 abgezogen und nach der Benutzung entsorgt und durch eine andere Einheit ersetzt.

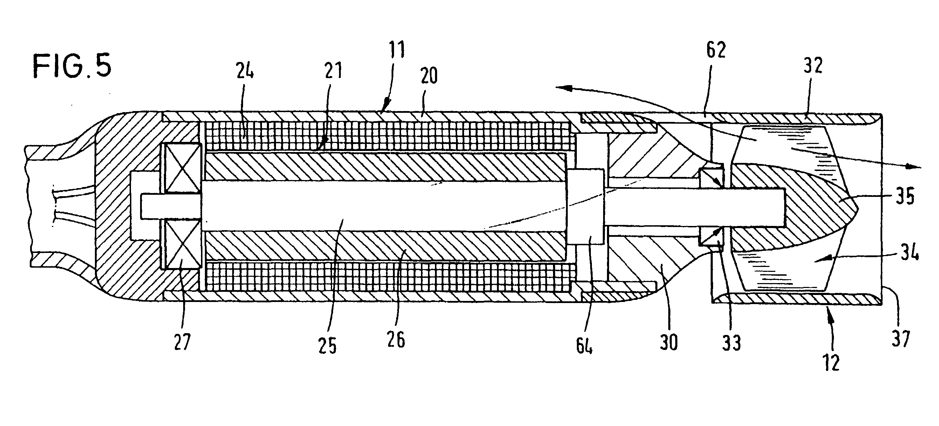

Das Ausführungsbeispiel von Fig. 5 entspricht weitgehend demjenigen der Fig. 2, wobei gleiche Teile mit gleichen Bezugszeichen versehen sind.

Gemäß Fig. 5 ist das Flügelrad 34 auf der rotierenden Welle 25 des Elektromotors 21 fest angebracht. Die Motorwelle 25 ragt durch das von dem Motorgehäuse 20 nach vorne abstehende Nabenteil 30 hindurch und ist an dem vorderen Ende des Nabenteils mit einem Lager 33 gelagert.

Das Pumpengehäuse 32 ist mit dem Motorgehäuse 20 durch einen in Längsrichtung verlaufenden Verbindungssteg 62 verbunden, der bündig mit der Wand des Motorgehäuses 20 nach vorne vorsteht und ebenfalls bündig in die Wand des Pumpengehäuses 32 übergeht. Auf diese Weise entfallen die radialen Rippen 31 in Fig. 2 und die Länge des Pumpengehäuses 32 kann kürzer ausgebildet werden als in Fig. 2. Anstelle eines einzigen Verbindungssteges 62 können auch mehrere Verbindungsstege mit gegenseitigem Umfangsabstand vorgesehen sein.

Damit die Motorwelle 25 sich im Motorgehäuse 20 nicht axial verschiebt, ist sie mit einem radial abstehenden Kragen 64 versehen, der sich mit seiner Rückseite an dem Rotor 26 abstützt und mit seiner Vorderseite gegen das Nabenteil 30 stoßen kann, so daß ein Herausziehen der Motorwelle aus dem Motorgehäuse verhindert wird.

## Patentansprüche

1. Intravasale Blutpumpe mit einem Antriebsteil (11), der in einem Motorgehäuse (20) einen Elektromotor (21) enthält und mit einem Katheter (14) verbunden ist, und einem Pumpenteil (12), der ein auf einer Motorwelle (25) sitzendes, in einem rohrförmigen Pumpengehäuse (32) drehbares Flügelrad (34) enthält,
wobei das Motorgehäuse (20) und das Pumpengehäuse (32) im Wesentlichen gleiche Durchmesser haben und koaxial in axialem Abstand voneinander angeordnet sind und die Motorwelle (25) durch ein von dem Motorgehäuse (20) abstehendes stationäres Nabenteil (30) verläuft, welches das Pumpengehäuse (32) trägt,
und wobei die Motorwelle (25) in genau zwei Lagern, gelagert ist, von denen eines (27) an einem Ende des Motorgehäuses (20) angeordnet ist,
**dadurch gekennzeichnet,**
**dass** das zweite Lager (33) am flügelradseitigen Ende des stationären Nabenteils (30) angeordnet ist.

2. Intravasale Blutpumpe nach Anspruch 1, **dadurch gekennzeichnet, dass** das stationäre Nabenteil (30) abstehende Rippen (31) zum Halten des Pumpengehäuses (32) aufweist.

3. Intravasale Blutpumpe nach Anspruch 1, **dadurch gekennzeichnet, dass** das Pumpengehäuse (32) mit dem Motorgehäuse (20) über mindestens einen achsparallel verlaufenden Verbindungssteg (62) verbunden ist.

4. Intravasale Blutpumpe nach Anspruch 1 oder 2, **dadurch gekennzeichnet, daß** das flügelradseitige Lager (33) zugleich als Wellendichtung ausgebildet ist.

5. Intravasale Blutpumpe nach einem der Ansprüche 1-4, **dadurch gekennzeichnet, daß** das Pumpengehäuse (32), das Flügelrad (34) und der Schaft (25) eine Wegwerfeinheit (60) bilden, die als Ganzes von dem Motorgehäuse (20) abziehbar ist.

6. Intravasale Blutpumpe nach Anspruch 5, **dadurch gekennzeichnet, daß** zu der Wegwerfeinheit auch der Rotor (26) des Motors (21) gehört.

## Claims

1. An intravascular blood pump comprising a drive unit (11) including an electric motor (21) in a motor housing (20) and being connected to a catheter (14), and a pump unit (12) including an impeller (34) seated on a motor shaft (25) and arranged for rotation in a tubular pump housing (32),
the motor housing (20) and the pump housing (32) having substantially the same diameter and being arranged coaxillay at an axial distance from each other, and the motor shaft (25) extending through a stationary hub member (30) projecting from the motor housing (20) and supporting the pump housing (32),
and the motor shaft (25) being supported in exactly two bearings of which one (27) is arranged on one end of the motor housing (20),
**characterized in**
the second bearing (33) is arranged on the impeller-side end of the stationary hub member (30).

2. The intravascular blood pump according to claim 1, **characterized in that** the stationary hub member (30) comprises projecting ribs (31) for holding the pump housing (32).

3. The intravascular blood pump according to claim 1, **characterized in that** the pump housing (32) is connected to the motor housing (20) by at least one axis-parallel connecting web (62).

4. The intravascular blood pump according to claim 1, **characterized in that** the impeller-side bearing (33) is further designed as a shaft sealing.

5. The intravascular blood pump according to any one of claims 1-4, **characterized in that** the pump housing (32), the impeller (34) and the shaft (25) form a disposable unit (60) which can be withdrawn as one unit from the motor housing (20).

6. The intravascular blood pump according to claim 6, **characterized in that** the disposable unit includes also the rotor (26) of the motor (21).

## Revendications

1. Pompe à sang intravasculaire comportant une partie entraînement (11) qui renferme un moteur électrique (21) dans un carter de moteur (10) et est reliée à un cathéter (14) ainsi qu'une partie pompe qui renferme une roue à palettes (34) s'appuyant sur un arbre moteur (25) et pouvant tourner dans un carter de pompe (32) tubulaire,
dans laquelle le carter de moteur (20) et le carter de pompe (32) ont essentiellement le même diamètre et sont disposés de façon coaxiale à distance axiale l'un de l'autre et l'arbre moteur (25) traverse un moyeu stationnaire (30) faisant saillie par rapport au carter de pompe (32),
et dans laquelle l'arbre moteur (25) se loge dans exactement deux paliers dont un (27) est disposé à une extrémité du carter de moteur (20),
**caractérisé en ce que**
le deuxième palier (33) est disposé à l'extrémité du moyeu stationnaire (30) du côté de la roue à palettes.

2. Pompe à sang intravasculaire selon la revendication 1, **caractérisée en ce que** le moyeu stationnaire (30) présente des nervures saillantes (31) pour tenir le carter de pompe (32).

3. Pompe à sang intravasculaire selon la revendication 1, **caractérisée en ce que** le carter de pompe (32) est relié au carter de moteur (20) par au moins une patte de raccordement (62) s'étendant parallèlement à l'axe.

4. Pompe à sang intravasculaire selon la revendication 1 ou 2, **caractérisée en ce que** le palier (33) qui se trouve côté roue à palettes est simultanément configuré en tant que joint étanche pour l'arbre.

5. Pompe à sang intravasculaire selon l'une des revendications 1 à 4, **caractérisée en ce que** le carter de pompe (32), la roue à palettes (34) et l'arbre (25) forment une unité à usage unique (60) que l'on peut retirer d'un seul tenant du carter de moteur (20).

6. Pompe à sang intravasculaire selon la revendication 5, **caractérisée en ce que** le rotor (26) du moteur (21) fait également partie de l'unité à usage unique.
